# EUROPÄISCHE PATENTSCHRIFT

(11) **EP 3 393 543 B1**
(45) Veröffentlichungstag und Bekanntmachung des Hinweises auf die Patenterteilung: **14.08.2019**
(21) Anmeldenummer: 18704228.8
(22) Anmeldetag: 08.02.2018
(51) Int. Cl.: A61M 1/10, A61M 1/12

(54) **KATHETERPUMPE MIT EINEM PUMPENKOPF ZUM EINFÜHREN IN DAS ARTERIELLE GEFÄSSSYSTEM**
CATHETER PUMP WITH PUMP HEAD FOR INTRODUCTION IN ARTERIAL VESSEL SYSTEM
POMPE À CATHÉTER AVEC EMBOUT DE POMPE POUR INTRODUCTION DANS LE SYSTÈME DE VAISSEAUX ARTÉRIELLES

(30) Priorität: 13.02.2017 DE 102017102828
(43) Veröffentlichungstag der Anmeldung: 31.10.2018
(73) Patentinhaber: Cardiobridge GmbH, 72379 Hechingen (DE)
(72) Erfinder: EPPLE, Klaus, 72414 Rangendingen (DE)
(74) Vertreter: DREISS Patentanwälte PartG mbB
(86) Internationale Anmeldenummer: PCT/EP2018/053125
(87) Internationale Veröffentlichungsnummer: WO 2018/146173

(56) Entgegenhaltungen:
- EP-B1- 0 768 900
- EP-B1- 2 308 422
- WO-A2-03/103745

## Beschreibung

Die Erfindung betrifft eine Katheterpumpe mit einem Pumpenkopf zum Einführen in das arterielle Gefäßsystem wie zum Beispiel die Aorta oder das Herz, mit einem Außenkatheter, einen im Außenkatheter angeordneten Innenkatheter und einer im Innenkatheter verdrehbar angeordneten Rotorwelle zum Antreiben eines am Pumpenkopf vorgesehen, expandierbaren Förderelements, wobei das Förderelement zwischen einer distalen Lagerstelle und einer proximalen Lagerstelle drehgelagert ist, wobei der Außenkatheter an seinem distale Ende einen die proximale Lagerstelle umgebenden Hülsenabschnitt aufweist, und wobei die proximale Lagerstelle zum Expandieren des Förderelements relativ zum Hülsenabschnitt in axialer Richtung bewegbar ist.

Derartige Katheterpumpen sind beispielsweise aus der EP 2 308 422 B1 bekannt. Als rotierendes Förderelement kann beispielsweise, wie in der EP 768 900 B1 beschrieben, ein Rotor mit ausklappbaren Propellern Verwendung finden. Ebenso ist denkbar, dass anders ausgebildete Förderelemente, wie beispielsweise helixartig ausgebildete Wendel, Verwendung finden können.

Katheterpumpen werden als temporäres Kreislaufunterstützungssystem in die Aorta von Patienten eingesetzt, insbesondere dann, wenn das natürliche Herz nicht in der Lage ist, den Körper mit ausreichend Sauerstoff versetztem Blut zu versorgen. Das Förderelement und die Rotorwelle werden dabei mit vergleichsweise hohen Drehzahlen im Bereich von 7000 bis 15000 Drehungen pro Minute betrieben. Der Pumpenkopf der Katheterpumpe kann insbesondere nach einer Operation mehrere Tage in der Aorta verbleiben.

Der vorliegenden Erfindung liegt die Aufgabe zugrunde, die proximale Lagerstelle der eingangs genannten Katheterpumpe vorteilhaft auszubilden.

Diese Aufgabe wird durch eine Katheterpumpe mit den Merkmalen des Patentanspruchs 1 gelöst. Die Erfindung sieht folglich vor, dass die proximale Lagerstelle, also die Lagerstelle, die in axialer Richtung zwischen den Kathetern und der anderen, distalen Lagerstelle liegt, eine Lageraufnahme umfasst, die eine Drehlagerstelle für einen mit dem distalen Ende der Rotorwelle drehfest verbundenen Drehkopf und eine dazu axial beabstandete Krafteinleitstelle für einen am distalen Ende des Innenkatheters vorgesehenen Krafteinleitabschnitt zum axialen Bewegen der proximalen Lagerstelle relativ zum Hülsenabschnitt aufweist. Durch Vorsehen einer solchen Lageraufnahme, die die Drehlagerstelle und die Krafteinleitstelle aufweist, können mehrere Funktionen in einem Bauteil auf kleinstem Bauraum realisiert werden. Zum einen kann das Förderelement, und insbesondere ein Rotor des Förderelements, in der Drehlagerstelle drehsicher gelagert werden. Zum anderen kann aufgrund des Vorsehens der Krafteinleitstelle an der Lageraufnahme eine Relativbewegung zwischen dem Hülsenabschnitt des Außenkatheders und der Lageraufnahme zum Expandieren des Förderelements funktionssicher erfolgen.

Der Hülsenabschnitt kann dabei einstückig am Außenkatheter angeordnet sein oder von diesem gebildet werden. Ferner ist denkbar, dass der Hülsenabschnitt als separates Bauteil ausgebildet sein kann, das fest mit dem Außenkatheter verbunden ist.

Zum Kontrahieren oder Einklappen des Förderelements wird der Hülsenabschnitt relativ zur proximalen Lagerstelle in axialer Richtung weg von der distalen Lagerstelle bewegt.

Vorteilhafterweise ist die Lageraufnahme hülsenartig ausgebildet. Dabei werden die Drehlagerstelle von einer ersten Innennut und die Krafteinleitstelle von einer zweiten Innennut gebildet. Insgesamt kann dadurch eine vergleichsweise einfach aufgebaute Lageraufnahme mit der Drehlagerstelle und der Krafteinleitstelle gebildet werden. Die erste Innennut kann dabei vergleichsweise flach ausgebildete Anfasungen aufweisen, so dass der Drehkopf entsprechend günstig gelagert werden kann. Die zweite Innennut kann dabei insbesondere so ausgebildet werden, dass ein als Ringbund ausgebildeter Krafteinleitabschnitt funktionssicher von der zweiten Innennut aufgenommen werden kann.

Eine besonders bevorzugte Ausführungsform sieht vor, dass die Lageraufnahme wenigstens zwei Lagerschalen umfasst. Beim Vorsehen von zwei Lagerschalen finden also zwei Halbschalen Verwendung. Die Verwendung von zwei oder mehr Lagerschalen hat den Vorteil, dass eine Montage der proximalen Lagerstelle auf einfache Art und Weise möglich ist.

Dazu ist der Hülsenabschnitt vorteilhafterweise so ausgebildet, dass zur Montage der proximalen Lagerstelle zunächst die Lagerschalen in radialer Richtung auf den Drehkopf und auf den Krafteinleitabschnitt aufgesetzt werden und anschließend der Hülsenabschnitt in axialer Richtung über die Lagerschalen geschoben wird. Dies hat den Vorteil, dass weitere Montage- oder Befestigungsmittel entfallen können. Die Lagerschalen sind sicher im Hülsenabschnitt angeordnet.

Die Lageraufnahme und insbesondere die Lagerschalen können vorzugsweise auf der dem Hülsenabschnitt zugewandten, radialen Außenseite Vertiefungen derart aufweisen, dass zwischen dem Innenkatheter und dem Außenkatheter hindurchströmende Spülflüssigkeit zwischen der Lageraufnahme, bzw. den Lagerschalen, und der Innenwandung des Hülsenabschnitts hindurchströmen kann. Die Vertiefungen sind dabei vorzugsweise als in axialer Richtung verlaufende Rillen in die Lageraufnahme bzw. die Lagerschalen eingeformt. Dadurch kann gewährleistet werden, dass Spülflüssigkeit, welche zwischen dem Außenkatheter und dem Innenkatheter hin zum Pumpenkopf gefördert wird, die proximale Lagerstelle passieren kann, ohne dass die Flüssigkeit die Drehlagerstelle passieren muss.

Ferner ist erfindungsgemäß denkbar, dass sich an das distale Ende der Lageraufnahme ein Stauraum anschließt, in dem sich im Betrieb Spülflüssigkeit sammelt und von dem aus die Spülflüssigkeit weiter zur distalen Lagerstelle, in die Aorta und/oder zurück durch den Innenkatheter geleitet wird. Hierdurch kann erreicht werden, dass zum einen auch die distale Lagerstelle mit entsprechender Spülflüssigkeit versorgt wird. Zum anderen kann ein Teil der Spülflüssigkeit, der im Bereich von einem Drittel der eingebrachten Spülflüssigkeit liegen kann, vorbei am Drehkopf durch die Drehlagerstelle hindurch zurück durch den Innenkatheter abgeführt werden. Dadurch kann erreicht werden, dass auch die Drehlagerstelle ausreichend mit entsprechender Spülflüssigkeit gespült und geschmiert werden kann. Die die Drehlagerstelle passierende Spülflüssigkeit wird also zurück durch den Innenkatheter bzw. durch ein Lumen zwischen dem Innenkatheter und der Rotorwelle geführt, so dass ein möglicher Lagerabrieb möglichst nicht in die Blutbahn des Patienten gelangt.

Der Krafteinleitabschnitt ist vorteilhafterweise an einer Buchse angeordnet, die fest am distalen Ende des Innenkatheters angeordnet ist. Die Buchse kann dabei den Innenkatheter in radialer Richtung umgeben. Der Krafteinleitabschnitt kann insbesondere als in radialer Richtung abstehender Ringbund ausgebildet sein, der in die zweite Innennut der Lageraufnahme eingreift. Hierdurch kann eine sichere in axialer Richtung wirkende Bewegungskopplung zwischen dem Innenkatheter bzw. der Buchse und der Lageraufnahme, bzw. der Lagerschalen, bereitgestellt werden.

Die Buchse kann ihrerseits auf der der Drehlagerstelle abgewandten Seite einen zum Krafteinleitabschnitt beabstandeten Bundabschnitt aufweisen. Dieser Bundabschnitt kann als axialer Anschlag gegen den Außenkatheter, bzw. gegen eine zwischen der Buchse und dem Außenkatheter vorgesehene Distanzhülse Verwendung finden.

Dabei ist vorteilhaft, wenn die Distanzhülse in radialer Richtung verlaufende Ausnehmungen zur Durchleitung von Spülflüssigkeit aufweist. Dadurch kann gewährleistet werden, dass Spülflüssigkeit, die zwischen dem in den Katheter und dem Außenkatheter hin zur proximalen Lagerstelle gefördert wird, auch dann zwischen dem Unterabschnitt und der Distanzhülse hindurch geleitet werden kann, wenn die Distanzhülse am Bundabschnitt vollständig zum Anliegen kommt.

Weitere Einzelheiten und vorteilhafte Ausgestaltungen der Erfindung sind der nachfolgenden Beschreibung zu entnehmen, anhand derer ein Ausführungsbeispiel der Erfindung näher beschrieben und erläutert ist.

Es zeigen:
Figur 1 eine perspektivische Ansicht auf einen Pumpenkopf einer erfindungsgemäßen Katheterpumpe mit teilaufgeschnittener proximalen Lagerstelle;
Figur 2 einen Längsschnitt durch die in Figur 1 gezeigte proximale Lagerstelle und
Figur 3 die in Figur 2 gezeigte Lagerstelle vor deren Montage.

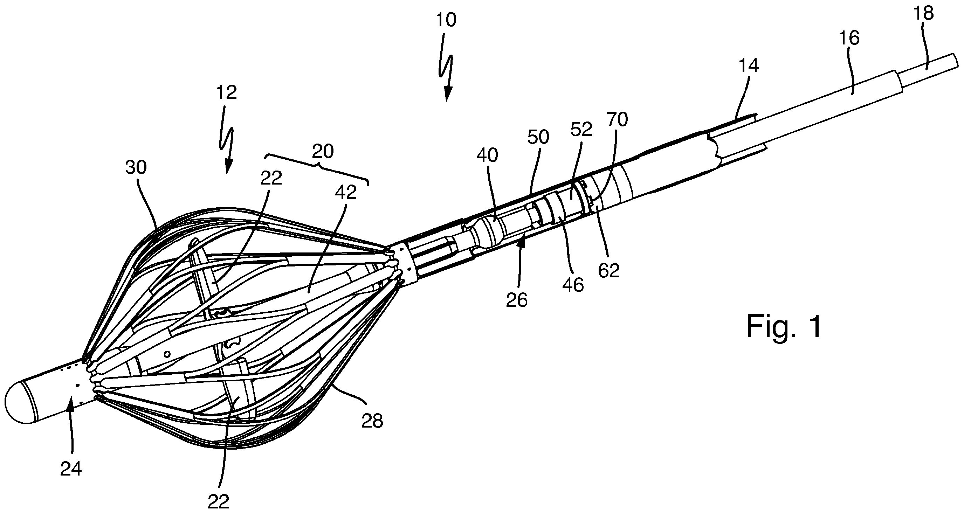

Die in der Figur 1 gezeigte Katheterpumpe 10 weist einen Pumpenkopf 12 zum Einführen in die Aorta oder das Herz eines Patienten auf. Die Pumpe 10 umfasst einen Außenkatheter 14, einen Innenkatheter 16 und eine im Innenkatheter 16 verdrehbar angeordnete Rotorwelle 18. Über die Rotorwelle 18 kann ein in Figur 1 expandiert dargestelltes Förderelement 20 in Form eines Rotors 42 mit Propellern 22 angetrieben werden. Die Propeller 22 sind dabei zwischen einer distalen Lagerstelle 24 und einer proximalen Lagerstelle 26 auf dem Rotor 42 angeordnet. Das Förderelement 20 bzw. die Propeller 22 werden von einem Käfig 28 umgeben, der verschiedene Filamente 30 vorsieht. Im expandierten Zustand, der in Figur 1 gezeigt ist, ist der Käfig 28 bauchartig ausgebildet, so dass die Propeller 22 innerhalb des Käfigs 28 frei drehen können. Zum Einführen des Pumpenkopfes 12 in die Aorta ist der Pumpenkopf 12 nicht expandiert, sondern in einem kollabierten bzw. eingeklappten Zustand. In diesem kollabierten Zustand liegen die Propeller 22 nahe an der Drehachse des Rotors 42 und die Filamente 30 des Käfigs 28 befinden sich in einer parallel zur Drehachse des Rotors 42 verlaufenden Position.

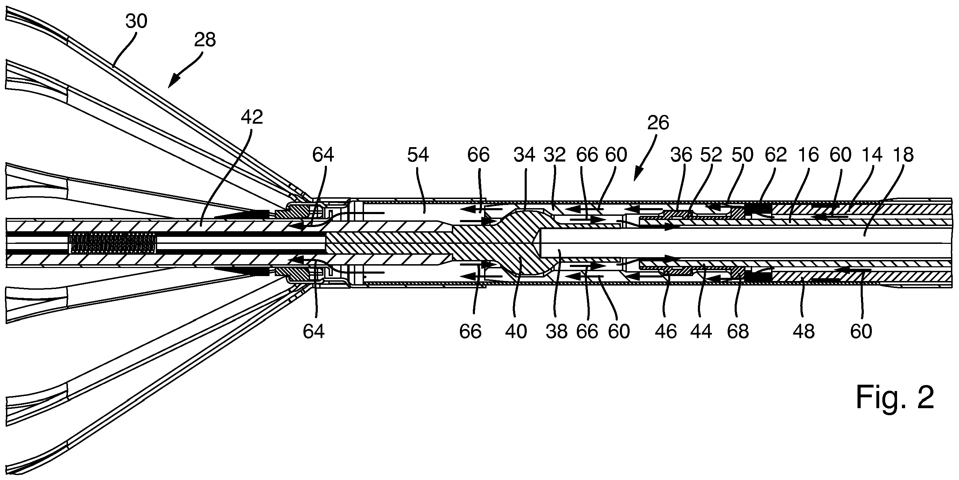

Wie aus dem Schnitt gemäß Figur 2 deutlich wird, weist die proximale Lagerstelle 26 eine Lageraufnahme 32 auf, die eine Drehlagerstelle 34 sowie eine Krafteinleitstelle 36 umfasst. Die Drehlagerstelle 34 sowie die Krafteinleitstelle 36 sind dabei jeweils als umlaufende Innennuten in die hülsenartig ausgebildete Lageraufnahme 32 eingebracht.

In der Drehlagerstelle 34 ist ein mit dem distalen Ende 38 der Rotorwelle 18 drehfest verbundener Drehkopf 40, vorzugsweise ein ein Kugelkopf, drehgelagert. Über den Drehkopf 40 wird der Rotor 42 mit den Propeller 22 angetrieben. Die Rotorwelle 18 ihrerseits wird im Betrieb an ihrem proximalen, nicht dargestellten Ende durch einen Antrieb angetrieben.

In der Krafteinleitstelle 36 ist ein Krafteinleitabschnitt 46 vorgesehen. Der Krafteinleitabschnitt 46 wird von einem Ringbund gebildet, der an einer mit dem Innenkatheter 16 fest verbundenen Buchse 52 vorgesehen ist. Der Ringbund greift dabei formschlüssig in die von der Lageraufnahme 32 gebildete, als umlaufende Innennut ausgebildete Krafteinleitstelle 36 ein.

Die Lageraufnahme 32 ihrerseits ist in einem am distalen Ende 48 des Außenkatheters 14 angeordneten Hülsenabschnitt 50 untergebracht, der in axialer Richtung relativ zur Lageraufnahme 32, und damit zum Innenkatheter 16 bewegbar ist.

Durch die beschriebene Anordnung kann durch axiales Bewegen des Außenkatheters 14 relativ zum Innenkatheter 16 und damit relativ zur mit dem Innenkatheder 16 bewegungsgekoppelten Lageraufnahme 32 der Hülsenabschnitt 50 aus der in der Figur 2 gezeigten distalen Position, in der das Förderelement 20 expandiert wird, nach rechts in eine proximale Position verschoben werden, wodurch die Propeller 22 eingeklappt werden und der Käfig 28 kollabiert.

In der in der Figur 2 gezeigten distalen Position des Hülsenabschnitts 50 befindet sich an das distale Ende der Lageraufnahme 32 angrenzend ein Stauraum 54. Dieser Stauraum 54 entsteht dadurch, dass der Hülsenabschnitt 50 beim Expandieren des Förderelements 20 axial in distaler Richtung verschoben wird. In der proximalen Position des Hülsenabschnitts 50, also bei kollabiertem Förderelement 20, befindet sich die Lageraufnahme 32 im Stauraum 54.

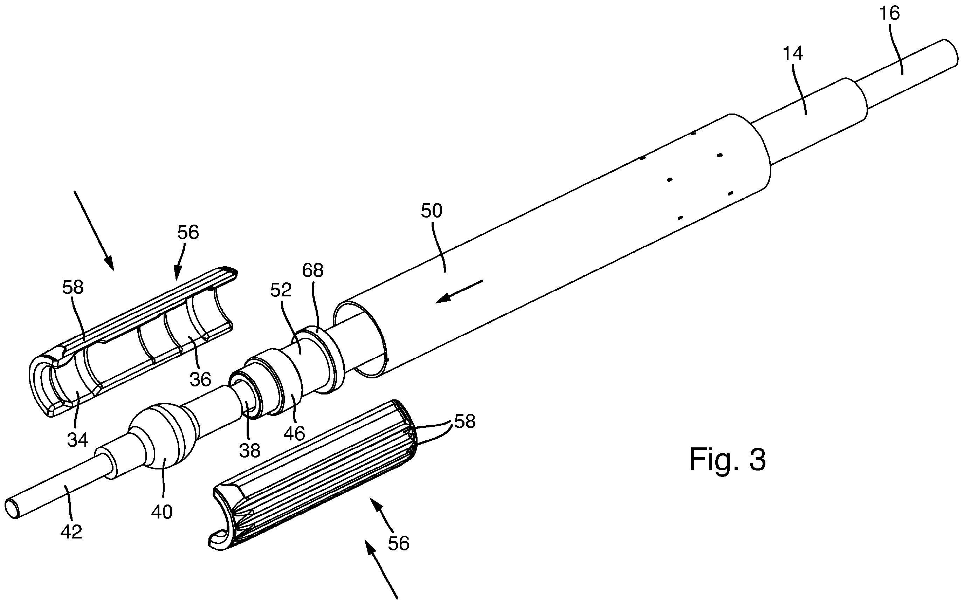

Wie aus Figur 3 deutlich wird, besteht die Lageraufnahme 32 aus zwei Lagerschalen 56. Zur Montage der proximalen Lagerstelle 26 werden zunächst die beiden Lagerschalen 56 in radialer Richtung auf den Drehkopf 40 und den Krafteinleitabschnitt 46 aufgesetzt. Daran anschließend wird der Hülsenabschnitt 50 in axialer Richtung über die Lagerschale 56 geschoben. Dies hat den Vorteil, dass eine Montage bzw. eine Sicherung der Lagerschalen 56 ohne weitere Bauteile oder Sicherungsmittel erfolgen kann. Mit dieser Anordnung können die Anforderungen an diese Lagerung auf kleinstem Bauraum realisiert werden.

Die Buchse 52 weist auf der der Drehlagerstelle 34 abgewandten Seite einen zum Krafteinleitabschnitt 46 beabstandeten Bundabschnitt 68 auf. Der Bundabschnitt 68 kann dabei zusammen mit einer Distanzhülse 62 als Axialanschlag dienen, und somit den axialen Verschiebeweg des Hülsenabschnitts 50 begrenzen.

Wie aus Figur 3 ferner deutlich wird, weisen die Lagerschalen 56 auf ihrer radial äußeren Seite Vertiefungen 58 in Form von sich in axialer Richtung erstreckenden Rillen auf. Im Betrieb der Katheterpumpe 10 kann dadurch zwischen dem Innenkatheter 16 und dem Außenkatheter 14 hindurchströmende Spülflüssigkeit, die in Figur 2 mit dem Pfeil 60 angedeutet ist, und die eine Distanzhülse 62 passiert, zwischen den Lagerschalen 56 und der Innenwandung des Hülsenabschnitts 50 in den Stauraum 54 einströmen. Die Spülflüssigkeit, die unter Druck zwischen den Innenkatheter 16 und den Außenkatheter 14 gepumpt wird, sammelt sich dadurch im Stauraum 54, so dass ein Großteil der Spülflüssigkeit im Zulauf nicht in Kontakt mit dem Drehkopf 40 oder der Drehlagerstelle 34 kommt. Vom Stauraum 54 aus kann die Spülflüssigkeit dann über entsprechende, in den Figuren nicht gezeigte Ausnehmungen, wie mit den Pfeilen 64 angedeutet, hin zur distalen Lagerstelle 24 zur Versorgung der Lagerstelle 24 transportiert werden. Ein weiterer Teil der Spülflüssigkeit kann aus dem Stauraum 54 über eine Drehentkoppelung, die in den Figuren nicht weiter bezeichnet ist, in die Aorta austreten. Ein dritter Teil, ca. ein Drittel der Spülflüssigkeit, kann vom Stauraum 54, wie mit den Pfeilen 66 angedeutet, die Drehlagerstelle 34 zu deren Spülung und Schmierung durchströmen und zwischen dem Innenkatheter 16 und der Rotorwelle 18 zum proximalen Ende des Außenkatheters 14 abgeführt werden. Hierdurch kann erreicht werden, dass zum einen der Drehkopf 40 und zum anderen die gesamte Rotorwelle 18 mit einer ausreichenden Spülung und Schmierung versorgt wird, wobei der Anteil der Spülflüssigkeit, der die Lageraufnahme 32 durchströmt, nicht in die Aorta des Patienten gelangt.

Die Buchse 52 weist auf der der Drehlagerstelle 34 abgewandten Seite eine zum Krafteinleitabschnitt 46 beabstandeten Bundabschnitt 68 auf. Dieser kann zusammen mit der Distanzhülse 62 als Axialanschlag dienen. Damit die Spülflüssigkeit ungehindert zwischen der Buchse 52 und der Distanzhülse 62 hindurch geleitet werden kann, weist die Distanzhülse 62, wie in Figur 1 dargestellt, axiale Ausnehmungen 70 auf, die auch dann offen bleiben, wenn die Distanzhülse 62 am Bundabschnitt 68 der Buchse 52 anliegt.

Durch den beschriebenen Aufbau der proximalen Lagerstelle 26 kann zum einen eine einfache und dennoch betriebssichere Montage der Lagerstelle 26 mit den beiden Lagerschalen 56 erfolgen, wobei dennoch ein axiales Bewegen der proximalen Lagestelle 26 relativ zum Hülsenabschnitt 50 zum Expandieren des Förderelements 20 gewährleistet ist.

## Patentansprüche

1. Katheterpumpe (10) mit einem Pumpenkopf (12) zum Einführen in das arterielle Gefäßsystem, mit einem Außenkatheter (14), einem im Außenkatheter (14) angeordneten Innenkatheter (16) und einer im Innenkatheter (16) verdrehbar angeordneten Rotorwelle (18) zum Antrieben eines am Pumpenkopf (12) vorgesehenen, expandierbaren Förderelements (20), wobei das Förderelement (20) zwischen einer distalen Lagerstelle (24) und einer proximalen Lagerstelle (26) drehgelagert ist, wobei der Außenkatheter (14) an seinem distale Ende einen die proximale Lagerstelle umgebenden Hülsenabschnitt (50) aufweist, und wobei die proximale Lagerstelle (26) zum Expandieren des Förderelements (20) relativ zum Hülsenabschnitt (50) in axialer Richtung bewegbar ist, **dadurch gekennzeichnet, dass** die proximalen Lagerstelle (26) eine Lageraufnahme (32) umfasst, die eine Drehlagerstelle (34) für einen mit dem distalen Ende (38) der Rotorwelle (18) drehfest verbundenen Drehkopf (40) und eine dazu axial beabstandete Krafteinleitstelle (36) für einen am distalen Ende (44) des Innenkatheters (16) vorgesehenen Krafteinleitabschnitt (46) zum axialen Bewegen der proximalen Lagerstellen (26) relativ zum Hülsenabschnitt (50) aufweist.

2. Katheterpumpe (10) nach Anspruch 1, **dadurch gekennzeichnet, dass** die Lageraufnahme (32) hülsenartig ausgebildet ist und die Drehlagerstelle (34) von einer ersten Innennut und die Krafteinleitstelle (36) von einer zweiten Innennut gebildet wird.

3. Katheterpumpe (10) nach Anspruch 1 oder2, **dadurch gekennzeichnet, dass** die Lageraufnahme (32) wenigstens zwei Lagerschalen (56) umfasst.

4. Katheterpumpe (10) nach Anspruch 3, **dadurch gekennzeichnet, dass** der Hülsenabschnitt (50) derart ausgebildet ist, dass zur Montage der proximalen Lagerstelle (26) zunächst die Lagerschalen (56) in radialer Richtung auf den Drehkopf (40) und auf den Krafteinleitabschnitt (46) aufgesetzt werden und anschließend der Hülsenabschnitt (50) in axialer Richtung über die Lagerschalen (56) geschoben wird.

5. Katheterpumpe (10) nach einem der vorhergehenden Ansprüche, **dadurch gekennzeichnet, dass** die Lageraufnahme (32) auf der dem Hülsenabschnitt (50) zugewandten Außenseite Vertiefungen (58) derart aufweist, dass zwischen dem Innenkatheter (16) und dem Außenkatheter (14) hindurchströmende Spülflüssigkeit zwischen der Lageraufnahme (32) und der Innenwandung des Hülsenabschnitt (50) hindurchströmen kann.

6. Katheterpumpe (10) nach einem der vorhergehenden Ansprüche, **dadurch gekennzeichnet, dass** sich an das distalen Ende der Lageraufnahme (32) ein Stauraum (54) anschließt, in dem sich im Betrieb Spülflüssigkeit sammelt und von dem aus die Spülflüssigkeit weiter zur distalen Lagerstelle (24), in das arterielle Gefäßsystem und/oder zurück durch den Innenkatheter (16) geleitet wird.

7. Katheterpumpe (10) nach einem der vorhergehenden Ansprüche, **dadurch gekennzeichnet, dass** der Krafteinleitabschnitt (46) an einer Buchse (52) angeordnet ist, die fest am distalen Ende (44) des Innenkatheters (16) angeordnet ist.

8. Katheterpumpe (10) nach Anspruch 7, **dadurch gekennzeichnet, dass** die Buchse (52) auf der der Drehlagerstelle (34) abgewandten Seite einen zum Krafteinleitabschnitt (46) beabstandeten Bundabschnitt (68) aufweist, der zusammen mit einer Distanzhülse (62) einen axialen Anschlag bildet.

9. Katheterpumpe (10) nach Anspruch 8, **dadurch gekennzeichnet, dass** die Distanzhülse (62) in radialer Richtung verlaufende Ausnehmungen (70) zur Durchleitung von Spülflüssigkeit aufweist.

## Claims

1. A catheter pump (10) comprising a pump head (12) for introducing into the arterial vasculature, an outer catheter (14), an inner catheter (16) arranged in said outer catheter (14), and a rotor shaft (18) rotatably arranged in said inner catheter (16) for driving an expandable conveying element (20) provided at said pump head (12), wherein said conveying element (20) is rotatably mounted between a distal bearing point (24) and a proximal bearing point (26), wherein said outer catheter (14) has a sleeve portion (50) surrounding said proximal bearing point on the distal end thereof, and wherein said proximal bearing point is movable in the axial direction relative to said sleeve portion (50) in order to expand said conveying element (20), **characterized in that** said proximal bearing point (26) comprises a bearing receptacle (32) having a rotational bearing point (34) for a rotary head (40) connected to the distal end (38) of said rotor shaft (18) in a rotationally fixed manner and a force application point (36) axially spaced therefrom for a force application portion (46) provided at the distal end (44) of said inner catheter (16) for axially moving said proximal bearing points (26) relative to said sleeve portion (50).

2. The catheter pump (10) according to claim 1, **characterized in that** said bearing receptacle (32) is sleeve-shaped, said rotational bearing point (34) is formed by a first inner groove and said force application point (36) is formed by a second inner groove.

3. The catheter pump (10) according to claim 1 or 2, **characterized in that** said bearing receptacle (32) comprises at least two bearing shells (56).

4. The catheter pump (10) according to claim 3, **characterized in that** said sleeve portion (50) is formed such that, for mounting said proximal bearing point (26), first said bearing shells (56) are placed in the radial direction on said rotary head (40) and on said force application portion (46), and then said sleeve portion (50) is pushed in the axial direction over said bearing shells (56).

5. The catheter pump (10) according to any one of the preceding claims, **characterized in that** said bearing receptacle (32) has recesses (58) on the outside facing said sleeve portion (50) such that flushing liquid flowing between said inner catheter (16) and said outer catheter (14) can flow between said bearing receptacle (32) and the inner wall of said sleeve portion (50).

6. The catheter pump (10) according to any one of the preceding claims, **characterized in that** the distal end of said bearing receptacle (32) is adjoined by a storage space (54) in which flushing liquid is collected during operation and from which said flushing liquid is further passed to said distal bearing point (24), into the arterial vasculature and/or back through said inner catheter (16).

7. The catheter pump (10) according to any one of the preceding claims, **characterized in that** said force application portion (46) is arranged on a bushing (52) fixedly arranged on the distal end (44) of said inner catheter (16).

8. The catheter pump (10) according to claim 7, **characterized in that** said bushing (52) includes a collar portion (68) spaced from said force application portion (46) on the side facing away from said rotational bearing point (34), said collar portion (68) along with a spacer sleeve (62) forming an axial stop.

9. The catheter pump (10) according to claim 8, **characterized in that** said spacer sleeve (62) has recesses (70) extending in the radial direction for the passage of flushing liquid.

## Revendications

1. Pompe à cathéter (10) pourvue d'une tête de pompe (12) à introduire dans le système vasculaire artériel, d'un cathéter extérieur (14), d'un cathéter intérieur (16) agencé dans le cathéter extérieur (14) et d'un arbre de rotor (18) agencé en rotation dans le cathéter intérieur (16) pour entraîner un élément de refoulement (20) expansible prévu sur la tête de pompe (12), dans laquelle l'élément de refoulement (20) est monté de manière à pouvoir tourner entre un point d'appui distal (24) et un point d'appui proximal (26), dans laquelle le cathéter extérieur (14) présente à son extrémité distale une partie manchon (50) entourant le point d'appui proximal, et dans laquelle le point d'appui proximal (26) peut se mouvoir dans la direction axiale par rapport à la partie manchon (50) pour permettre l'expansion de l'élément de refoulement (20), **caractérisée en ce que** le point d'appui proximal (26) comporte un logement d'appui (32), qui présente un point d'appui en rotation (34) pour une tête rotative (40) reliée de manière solidaire en rotation à l'extrémité distale (38) de l'arbre de rotor (18) et un point d'application de force (36) espacé axialement de celui-ci pour une partie d'application de force (46) prévue à l'extrémité distale (44) du cathéter intérieur (16), pour le mouvement axial des points d'appui proximaux (26) par rapport à la partie manchon (50).

2. Pompe à cathéter (10) selon la revendication 1, **caractérisée en ce que** le logement d'appui (32) est du type manchon et le point d'appui en rotation (34) est formé par une première rainure intérieure et le point d'application de force (36) par une deuxième rainure intérieure.

3. Pompe à cathéter (10) selon la revendication 1 ou 2, **caractérisée en ce que** le logement d'appui (32) comporte au moins deux coquilles d'appui (56).

4. Pompe à cathéter (10) selon la revendication 3, **caractérisée en ce que** la partie manchon (50) est réalisée de telle manière que, pour le montage du point d'appui proximal (26), les coquilles d'appui (56) sont tout d'abord mises en place dans la direction radiale sur la tête rotative (40) et sur la partie d'application de force (46) et la partie manchon (50) est ensuite glissée sur les coquilles d'appui (56) dans la direction axiale.

5. Pompe à cathéter (10) selon l'une quelconque des revendications précédentes, **caractérisée en ce que** le logement d'appui (32) présente sur la face extérieure tournée vers la partie manchon (50) des évidements (58), de telle manière qu'un liquide de lavage circulant entre le cathéter intérieur (16) et le cathéter extérieur (14) peut circuler entre le logement d'appui (32) et la paroi intérieure de la partie manchon (50).

6. Pompe à cathéter (10) selon l'une quelconque des revendications précédentes, **caractérisée en ce qu'**un espace de retenue (54), dans lequel le liquide de lavage s'accumule lors du fonctionnement et à partir duquel le liquide de lavage continue d'être guidé en direction du point d'appui distal (24), dans le système vasculaire artériel et/ou à nouveau à travers le cathéter intérieur (16), se raccorde à l'extrémité distale du logement d'appui (32).

7. Pompe à cathéter (10) selon l'une quelconque des revendications précédentes, **caractérisée en ce que** la partie d'application de force (46) est agencée sur un coussinet (52) qui est agencé de manière fixe à l'extrémité distale (44) du cathéter intérieur (16).

8. Pompe à cathéter (10) selon la revendication 7, **caractérisée en ce que** le coussinet (52) présente sur la face opposée au point d'appui en rotation (34) une partie épaulement (68) qui est espacée de la partie d'application de force (46) et qui forme une butée axiale conjointement avec un manchon d'écartement (62).

9. Pompe à cathéter (10) selon la revendication 8, **caractérisée en ce que** le manchon d'écartement (62) présente des creux (70) s'étendant dans la direction radiale et permettant de faire passer le liquide de lavage.
